(19) Europäisches Patentamt
European Patent Office
Office européen des brevets

(11) Veröffentlichungsnummer : **0 059 167**
**B1**

(12) # EUROPÄISCHE PATENTSCHRIFT

(45) Veröffentlichungstag der Patentschrift : **24.04.85**

(51) Int. Cl.[4] : **C 07 C 79/35**, C 07 C 76/02, A 01 N 37/00

(21) Anmeldenummer : **82810069.3**

(22) Anmeldetag : **15.02.82**

(54) **3-Halogenalkoxy-4-nitro-2'-chlor-4'-trifluormethyl-diphenyläther, Verfahren zu deren Herstellung, sie enthaltende Mittel sowie deren Verwendung als Herbizide.**

(30) Priorität : **19.02.81 CH 1106/81**

(43) Veröffentlichungstag der Anmeldung : **01.09.82 Patentblatt 82/35**

(45) Bekanntmachung des Hinweises auf die Patenterteilung : **24.04.85 Patentblatt 85/17**

(84) Benannte Vertragsstaaten : **AT BE CH DE FR GB IT LI NL**

(56) Entgegenhaltungen :
**DE-A- 2 440 868**
**FR-A- 2 295 945**
**Die Akte enthält technische Angaben, die nach dem Eingang der Anmeldung eingereicht wurden und die nicht in dieser Patentschrift enthalten sind.**

(73) Patentinhaber : **CIBA-GEIGY AG**
**Postfach**
**CH-4002 Basel (CH)**

(72) Erfinder : **Rohr, Otto, Dr.**
**Kilbertweg 19**
**CH-4106 Therwil (CH)**

**EP 0 059 167 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue selektiv-herbizid wirksame 3-Halogenalkoxy-4-nitro-2'-chlor-4'-trifluormethyl-diphenyläther, deren Herstellung, sie als Wirkstoff enthaltende Mittel sowie deren Verwendung zur selektiven Unkrautbekämpfung in Kulturen von Getreide, Reis, Baumwolle und insbesondere Mais und Soja.

Herbizid wirksame Diphenyläther sind schon lange bekannt und z. T. im Handel erhältlich. 2'-Chlor-4-nitro-diphenyläther sind beispielsweise beschrieben in der US-Patentschrift 4 220 468 und in der japanischen Patentveröffentlichung 75 69 227.

Solche Verbindungen sind als Herbizide vorgeschlagen und praktisch verwendet worden, um zur Erleichterung der Landarbeit beizutragen und die Produktivitäten von landwirtschaftlichen Nutzpflanzen und Gartenbaunutzpflanzen zu verbessern.

Es besteht aber immer noch ein Bedürfnis, neue Herbizide zu finden, welche überlegene Herbizideigenschaften aufweisen. Herbizide, die für landwirtschaftliche und gartenbauliche Zwecke eingesetzt werden sollen, sind vorzugsweise Verbindungen, die bei Anwendung kleiner Dosismengen die Zielunkräuter selektiv bekämpfen, ohne dabei gegenüber Nutzpflanzen toxisch zu sein. Bekannte Herbizide weisen diese optimalen Herbizideigenschaften nicht immer auf.

Es wurde nun überraschenderweise gefunden, dass die neuen Wirkstoffe gemäss vorliegender Erfindung den in den Handel eingeführten Produkten und den strukturell nächst verwandten Verbindungen der Patentliteratur wie z. B. Verbindung Nr. 11 aus Tabelle 1 im US-Patent 4 220 468 und Verbindung Nr. 15 aus der japanischen Patentveröffentlichung 75 69 227 in der selektiven Unkrautbekämpfung in Kulturen von Getreide, Mais und Soja mit ihren vergesellschafteten Unkraütern überlegen sind.

Die erfindungsgemässen 3-Halogenalkoxy-4-nitro-2'-chlor-4'-trifluormethyl-diphenyläther entsprechen der allgemeinen Formel I

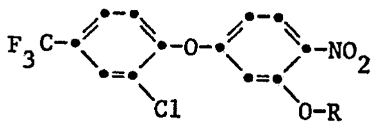

(I)

worin R Difluormethyl oder 2-Chlor-1,1,2-trifluoräthyl bedeutet.

Unter die Formel I fallen die folgenden Einzelverbindungen :

2'-Chlor-3-difluormethoxy-4'-trifluormethyl-4-nitro-diphenyläther und 3-(2-Chlor-1,1,2-trifluoräthoxy)-2'-chlor-4'-trifluormethyl-4-nitrodiphenyläther.

Zur Herstellung der neuen Diphenyläther der Formel I dienen die angeschlossenen Verfahren :

Nach einem ersten erfindungsgemässen Herstellungsverfahren erhält man die Verbindungen der Formel I, indem man das Phenoxyphenol der Formel II,

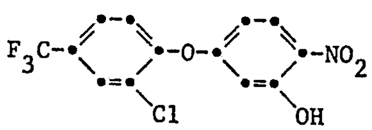

(II)

gegebenenfalls in Gegenwart einer Base, in einem inerten Lösungsmittel, mit einem Halogenalkan der Formel III umsetzt,

Hal—R (III)

worin R die unter Formel I angegebene Bedeutung hat und Hal Chlor, Brom oder Jod bedeutet.

Nach einem zweiten erfindungsgemässen Verfahren wird die Verbindung der Formel I, in der R den 2-Chlor-1,1,2-trifluoräthylrest bedeutet, hergestellt, indem man das Phenoxyphenol der Formel II in Gegenwart einer Base in einem inerten organischen Lösungsmittel, gegebenenfalls unter erhöhtem Druck, mit Chlortrifluoräthen umsetzt.

Das erste Verfahren wird mit Vorteil bei Temperaturen von 20° bis 150 °C, insbesondere zwischen 50° und 100 °C, bei Normaldruck durchgeführt. Als Lösungsmittel eignen sich Wasser oder Wasser-Gemische mit organischen Lösungsmitteln wie Aethern, z. B. Tetrahydrofuran, Dioxan ; Alkoholen, z. B. Methanol, Aethanol, i-Propanol ; Säureamiden, z. B. Dimethylformamid, 2-Pyrrolidinon, Hexamethylphosphorsäuretriamid ; Acetonitril oder Dimethylsulfoxid oder auch die genannten organischen Lösungsmittel selber. Geeignete Basen sind z. B. Hydroxide wie Natrium- und Kaliumhydroxid oder Hydride wie Natrium- oder Calciumhydrid.

Das zweite Verfahren wird vorteilhafterweise bei Temperaturen von 20° bis 150 °C, insbesondere zwischen 40° und 100 °C, unter Drücken von 1 bis 50 bar, vorzugsweise von 1 bis 12 bar ausgeführt. Geeignete polare, aprotische Lösungsmittel sind z. B. Dimethylformamid, 2-Pyrrolidinon, Hexamethylphosphorsäuretriamid, Acetonitril oder Dimethylfsulfoxid. Als Basen kommen beispielsweise in Frage Natrium- und Kaliumhydroxid, Natrium- und Calciumhydrid, Natriumamid oder Lithiumdiisopropylamid.

Die Ausgangsverbindung der Formel II ist in der US-PS 4 220 468 beschrieben, die der Formel III sind im Handel erhältlich.

Die erfindungsgemässen Verbindungen sind unter Normalbedingungen stabil. Ihre Handhabung bedarf keiner besonderen Vorsichtsmassnahmen.

Die Verbindungen der Formel I haben selektiv-herbizide Eigenschaften, die sie besonders geeignet erscheinen lassen zur Bekämpfung von mono- und dikotylen Unkräutern in Kulturen von Getreide, Reis, Baumwolle und insbesondere Mais und Soja.

Somit betrifft die Erfindung auch herbizide Mittel, welche einen neuen Wirkstoff der Formel I enthalten, sowie Verfahren zur pre- und post-emergenten Unkrautbekämpfung.

Die Verbindungen der Formel I werden in unveränderter Form oder vorzugsweise als Mittel zusammen mit den in der Formulierungstechnik üblichen Hilfsmitteln eingesetzt und werden daher z. B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z. B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d. h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle wie epoxidiertes Kokusnussöl oder Sojaöl; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbeserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z. B. Bimsstein, Ziegelbruch, Sepiolit oder Bentonit, als nicht sorptive Trägermaterialien z. B. Calcit oder Sand in Frage. Darüberhinaus kann eine Vielzahl von vorgranulierten Materialien anorganischeroder organischer Natur wie insbesonere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalz von höheren Fettsäuren ($C_{10}$-$C_{22}$), wie z. B. die Na- oder K-Salze der Oel- der Stearinsäure, oder von natürlichen Fettsäuregemischen, die z. B. aus Kokosnuss- oder Talgöl gewonnen werden können, genannt. Ferner sind auch die Fettsäure-methyltaurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einem Alkylrest mit 8 bis 22 C-Atomen auf, wobei Alkyl auch den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind z. B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z. B. Salze des Phosphorsäureesters eines p-Nonylphenol-(4-14)-Aethylenoxid-Adduktes in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die

3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslcihen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthoxyäthynol, Polyäthylenglykol und Octylphenoxypolyäthoxyäthanol erwähnt.

Ferner kommen auch Fettsäureester von Polyoxyäthylensorbitan wie das Polyoxyäthylensorbitantrioleat in Betracht.

Bei den kationischen Tensiden handelt es sich vor allem um quartäre Ammoniumsalze, welche als N-Substituenten mindestens eine Alkylrest mit 8 bis 22 C-Atomen enthalten und als weitere Substituenten niedrige, gegebenenfalls halogenierte Alkyl-, Benzyl- oder niedrige Hydroxylalkylreste aufweisen. Die Salze liegen vorzugsweise als Halogenide, Methylsulfate oder Aethylsulfate vor, z. B. das Stearyltrimethylammoniumchlorid oder das Benzyldi(2-chloräthyl)äthylammoniumbromid.

Die in der Formulierungstechnik gebräuchlichen Tenside sind u. a. in folgenden Publikationen beschrieben :

« Mc Cutcheon's Detergents and Emulsifiers Annual » MC Publishing Corp., Ridgewood, New Jersey, 1979.

Sisley and Wood, « Encyclopedia of Surface Active Agents », Chemical Publishing Co., Inc. New York, 1964.

Die pestiziden Zubereitungen enthalten in der Regel 0,1 bis 99 %, insbesondere 0,1 bis 95 %, Wirkstoff der Formel I, 1 bis 99 % eines festen oder flüssigen Zusatzstoffes und 0 bis 30 %, insbesondere 0,1 bis 25 % eines Tensides.

Insbesondere setzen sich bevorzugte Formulierungen folgendermassen zusammen : (% = Gewichtsprozent)

Lösungen

| | |
|---|---|
| Aktiver Wirkstoff : | 5 bis 95 %, vorzugsweise 10 bis 80 % |
| Lösungsmittel : | 95 bis 5 %, vorzugsweise 90 bis 0 % |
| oberflächenaktives Mittel : | 1 bis 30 %, vorzugsweise 2 bis 20 % |

Emulgierbare Konzentrate :

| | |
|---|---|
| Aktiver Wirkstoff : | 10 bis 50 %, bevorzugt 10 bis 40 % |
| oberflächenaktives Mittel : | 5 bis 30 %, vorzugsweise 10 bis 20 % |
| flüssiges Trägermittel : | 20 bis 95 %, vorzugsweise 40 bis 80 %. |

Stäube

| | |
|---|---|
| Aktiver Wirkstoff : | 0,5 bis 10 %, vorzugsweise 2 bis 8 % |
| festes Trägermittel : | 99,5 bis 90 %, vorzugsweise 98 bis 92 %. |

Suspensions-Konzentrate

| | |
|---|---|
| Aktiver Wirkstoff : | 5 bis 75 %, vorzugsweise 10 bis 50 % |
| Wasser : | 94 bis 25 %, vorzugsweise 90 bis 30 % |
| oberflächenaktives Mittel : | 1 bis 30 %, vorzugsweise 2 bis 25 %. |

Benetzbare Pulver

| | |
|---|---|
| Aktiver Wirkstoff : | 5 bis 90 %, vorzugsweise 10 bis 80 % und insbesondere 20 bis 60 % |
| oberflächenaktives Mittel : | 0,5 bis 20 %, vorzugsweise 1 bis 15 % |
| festes Trägermittel : | 5 bis 90 %, vorzugsweise 30 bis 70 %. |

Granulate

| | |
|---|---|
| Aktiver Wirkstoff : | 0,5 bis 30 %, vorzugsweise 3 bis 15 %, |
| festes Trägermittel : | 99,5 bis 70 %, vorzugsweise 97 bis 85 %. |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verünnt werden. Die Aufwandmengen betragen in der Regel 0,1 bis 10 kg AS/ha, vorzugsweise 0,25 bis 5 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

In den folgenden Beispielen sind die Temperaturen in Celsiusgraden °C, die Drücke in Millibar mb oder Bar b angegeben.

## Herstellungsbeispiele

### Beispiel 1

Herstellung von 2'-Chlor-3-difluormethoxy-4'-trifluormethyl-4-nitro-diphenyläther

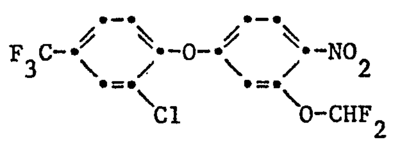

(Verbindung Nr. 1)

Zur Lösung von 33,4 g 2'-Chlor-3-hydroxy-4'-trifluormethyl-4-nitrodiphenyläther in 100 ml Dioxan werden unter kräftigem Rühren 15 ml 30 %ige wässrige Natriumhydroxidlösung zugesetzt und die Mischung auf 70° bis 75° erwärmt. Nachdem 1 Stunde lang gasförmiges Chlordifluormethan in die Reaktionslösung eingeleitet worden ist, wird die Lösung abgekühlt, mit 350 ml Wasser verdünnt und mit Aethylacetat extrahiert. Durch Eindampfen der organischen Phase erhält man 35,4 g 2'-Chlor-3-difluormethoxy-4'-trifluormethyl-4-nitrodiphenyläther als gelbes Oel. $n_D^{22}$ : 1,531 5.

$C_{14}H_7ClF_5NO_4$ (383,5)

ber.: C 43,83 %; H 1,84 %; N 3,65 %; F 24,76 %

gef.: C 43,8 %; H 2,0 %; N 3,8 %; F 24,6 %.

### Beispiel 2

Herstellung von 3-(2-Chlor-1,1,2-trifluoräthoxy)-2'-chlor-4'-trifluormethyl-4-nitro-diphenyläther.

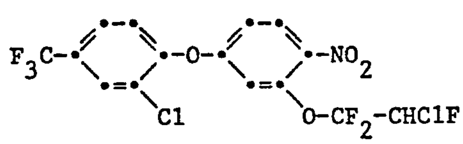

(Verbindung Nr. 2)

Zur Lösung von 33,4 g 2'-Chlor-3-hydroxy-4'-trifluormethyl-4-nitro-diphenyläther und 6,5 g Kaliumhydroxid in 100 ml Dimethylformamid in einem 300 ml Druckgefäss werden 25,4 g gasförmiges Chlortrifluoräthen zugepresst. Die Reaktionsmischung wird unter kräftigem Rühren für 8 Stunden auf 60° erwärmt. Der Druck erhöht sich dabei auf 10 b. Danach wird das Reaktionsgemisch in Eiswasser gegossen und mit Methylenchlorid extrahiert. Durch Eindampfen und Kugelrohrdestillation der organischen Phase erhält man 37 g 3-(2-Chlor-1,1,2-trifluoräthoxy)-2'-chlor-4'-trifluormethyl-4-nitro-diphenyläther als gelbes Oel. Spd. 150°/0,001 mb.

## Formulierungsbeispiele

### Beispiel 3

Formulierungsbeispiele für Wirkstoffe der Formel I (% = Gewichtsprozent)

| a) Emulsionskonzentrate | a) | b) | c) |
|---|---|---|---|
| Wirkstoff | 20 % | 40 % | 50 % |
| Ca-Dodecylbenzolsulfonat | 5 % | 8 % | 5,8 % |
| Ricinusöl-polyäthylenglykoläther (36 Mol AeO) | 5 % | — | — |
| Tributylphenoyl-polyäthylenglykoläther (30 Mol AeO) | — | 12 % | 4,2 % |
| Cyclohexanon | — | 15 % | 20 % |
| Xylolgemisch | 70 % | 25 % | 20 % |

Aus solchen Konzentraten können durch Verdünnen mit Wasser Emulsionen jeder gewünschten Konzentration hergestellt werden.

b) Lösungen

| | a) | b) | c) | d) |
|---|---|---|---|---|
| Wirkstoff | 80 % | 10 % | 5 % | 95 % |
| Aethylenglykol-monomethyl-äther | 20 % | — | — | — |
| Polyäthylenglykol M G 400 | — | 70 % | — | — |
| N-Methyl-2-pyrrolidon | — | 20 % | — | — |
| Epoxidiertes Kokosnussöl | — | — | 1 % | 5 % |
| Benzin (Siedegrenzen 160-190 °C) | — | — | 94 % | — |

Die Lösungen sind zur Anwendung in Form kleinster Tropfen geeignet.

c) Granulate

| | a) | b) |
|---|---|---|
| Wirkstoff | 5 % | 10 % |
| Kaolin | 94 % | — |
| Hochdisperse Kieselsäure | 1 % | — |
| Attapulgit | — | 90 % |

Der Wirkstoff wird in Methylenchlorid gelöst, auf den Träger aufgesprüht und das Lösungsmittel anschliessend im Vakuum abgedampft.

d) Stäubemittel

| | a) | b) |
|---|---|---|
| Wirkstoff | 2 % | 5 % |
| Hochdisperse Kieselsäure | 1 % | 5 % |
| Talkum | 97 % | — |
| Kaolin | — | 90 % |

Durch inniges Vermischen der Trägerstoffe mit dem Wirkstoff erhält man gebrauchsfertige Stäubemittel.

## Biologische Beispiele

### Beispiel 4

Pre-emergente Herbizid-Wirkung

Im Gewächshaus wird unmittelbar nach der Einsaat der Versuchspflanzen in Saatschalen die Erdoberfläche mit einer wässrigen Dispersion der Wirkstoffe, erhalten aus einem 25 %igen Emulsionskonzentrat behandelt. Es wurden Konzentrationen von 0,25-4 kg Wirksubstanz/Hektar angewendet. Die Saatschalen werden im Gewächshaus bei 22-25 °C und 50-70 % relativer Luftfeuchtigkeit gehalten und der Versuch nach 3 Wochen ausgewertet und die Resultate nach folgender Notenskala bonitiert :

1 = Pflanzen nicht gekeimt oder total abgestorben
2-3 = sehr starke Wirkung
4-6 = mittlere Wirkung
7-8 = geringe Wirkung
9 = keine Wirkung (wie unbehandelte Kontrolle).

| Wirkung | Verb.Nr. 1 | | | Verb.Nr. 2 | | |
|---|---|---|---|---|---|---|
| Aufwandmenge kg AS/ha | | | | | | |
| Testpflanze | 1 | 0,5 | 0,25 | 4 | 2 | 1 |
| Gerste | 7 | 9 | 9 | - | - | - |
| Weizen | 2 | 7 | 9 | 3 | 8 | 9 |
| Mais | 8 | 9 | 9 | 9 | 9 | 9 |
| Reis trocken | 4 | 4 | 7 | 7 | 9 | 9 |
| Avena fatua | 1 | 2 | 2 | 2 | 3 | 8 |

(Fortsetzung)

| Wirkung / Aufwandmenge kg AS/ha / Testpflanze | Verb.Nr. 1 | | | Verb.Nr. 2 | | |
|---|---|---|---|---|---|---|
| | 1 | 0,5 | 0,25 | 4 | 2 | 1 |
| Bromus tectorum | 1 | 2 | 2 | - | - | - |
| Lolium perenne | 1 | 1 | 2 | - | - | - |
| Alopecurus myos. | 1 | 1 | 2 | 1 | 1 | 1 |
| Digitaria sang. | 1 | 1 | 1 | - | - | - |
| Echinochloa c.g. | 1 | 1 | 1 | 1 | 3 | 8 |
| Sorghum halep. | 1 | 1 | 1 | - | - | - |
| Rottboellia ex. | 1 | 1 | 2 | 2 | 4 | 9 |
| Soja | 7 | 9 | 9 | 9 | 9 | 9 |
| Baumwolle | 8 | 9 | 9 | 2 | 3 | 9 |
| Abutilon | 1 | 1 | 1 | 1 | 1 | 3 |
| Sida spinosa | 1 | 1 | 1 | - | - | - |
| Amaranthus ret. | 1 | 1 | 1 | - | - | - |
| Chenopodium Sp. | 1 | 1 | 1 | 1 | 1 | 3 |
| Solanum nigrum | 1 | 1 | 1 | - | - | - |
| Chrysanthe. leuc. | 1 | 1 | 1 | - | - | - |
| Viola tricolor | 1 | 1 | 1 | 1 | 1 | 1 |
| Veronica Sp. | 1 | 1 | 1 | - | - | - |

## Beispiel 5

Post-emergente Herbizid-Wirkung (Kontaktherbizid)

Eine Anzahl Unkräuter, sowohl monokotyle wie dikotyle, wurden nach dem Auflaufen (in 4- bis 6-Blattstadium) mit einer wässrigen Wirkstoffdispersion in einer Dosierung von 0,5-2 kg Wirksubstanz pro Hektar auf die Pflanzen gespritzt und diese bei 24-26 °C und 45-60 % relativer Luftfeuchtigkeit gehalten. Mindestens 15 Tage nach der Behandlung wird der Versuch ausgewertet und das Ergebnis nach derselben Notenskala wie im pre-emergenten Versuch (Beispiel 4) bonitiert.

| Wirkung | Verb.Nr. 1 | | | Verb.Nr. 2 | | |
|---|---|---|---|---|---|---|
| Aufwandmenge kg AS/ha / Testpflanze | 2 | 1 | 0,5 | 2 | 1 | 0,5 |
| Weizen | 1 | 1 | 4 | 2 | 2 | 4 |
| Mais | 2 | 3 | 4 | 4 | 4 | 7 |
| Reis trocken | 3 | 4 | 4 | 4 | 5 | 6 |
| Avena fatua | 1 | 1 | 2 | 2 | 2 | 6 |
| Alopecurus myos. | 1 | 1 | 2 | 2 | 3 | 3 |

(Fortsetzung)

| Wirkung | Verb.Nr. 1 | | | Verb.Nr. 2 | | |
|---|---|---|---|---|---|---|
| Aufwandmenge kg AS/ha | 2 | 1 | 0,5 | 2 | 1 | 0,5 |
| Testpflanze | | | | | | |
| Echinochloa c.g. | 1 | 1 | 2 | 2 | 2 | 3 |
| Soja | 1 | 2 | 2 | 6 | 7 | 8 |
| Baumwolle | 1 | 1 | 2 | 1 | 1 | 1 |
| Abutilon | 1 | 1 | 1 | 1 | 1 | 1 |
| Xanthium Sp. | 1 | 1 | 4 | 2 | 2 | 3 |
| Chenopodium Sp. | 1 | 1 | 2 | 1 | 1 | 2 |
| Ipomoea | 1 | 1 | 1 | 1 | 1 | 1 |
| Sinapis | 1 | 1 | 1 | 1 | 2 | 2 |
| Galium aparine | 1 | 1 | 1 | 1 | 1 | 1 |
| Viola tricolor | 1 | 1 | 1 | 1 | 1 | 1 |

**Patentansprüche** (für die Vertragsstaaten : BE, CH, DE, FR, GB, IT, LI, NL)

1. 3-Halogenalkoxy-4-nitro-2'-chlor-4'-trifluormethyl-diphenyläther der allgemeinen Formel I,

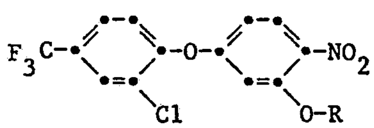

(I)

worin

R Difluormethyl oder 2-Chlor-1,1,2-trifluoräthyl bedeutet.

2. 3-(2-Chlor-1,1,2-trifluoräthoxy)-2'-chlor-4'-trifluormethyl-4-nitro-diphenyläther gemäss Anspruch 1.

3. 2'-Chlor-3-difluormethoxy-4'-trifluormethyl-4-nitro-diphenyläther gemäss Anspruch 1.

4. Verfahren zur Herstellung der Verbindungen der Formel I, gemäss Anspruch 1, dadurch gekennzeichnet, dass man das Phenoxyphenol der Formel II

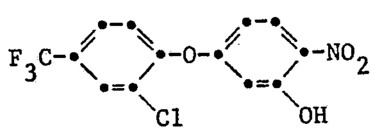

(II)

gegebenenfalls in Gegenwart einer Base, in einem inerten Lösungsmittel, mit einem Halogenalkan der Formel III umsetzt,

Hal—R (III)

worin R die unter Formel I angegebene Bedeutung hat und Hal für Chlor, Brom oder Jod steht.

5. Verfahren zur Herstellung der Verbindung der Formel I, gemäss Anspruch 1, in der R den 2-Chlor-1,1,2-trifluoräthylrest bedeutet, dadurch gekennzeichnet, dass man das Phenoxyphenol der Formel II in Gegenwart einer Baser in einem inerten organischen Lösungsmittel, gegebenenfalls unter erhöhtem Druck, mit Chlortrifluoräthan umsetzt.

6. Herbizides Mittel, dadurch gekennzeichnet, dass es neben Träger- und/oder anderen Zuschlagstoffen als Wirkstoff mindestens eine Verbindung gemäss Anspruch 1 enthält.

7. Verfahren zur Bekämpfung unerwünschten Pflanzenwuchses, dadurch gekennzeichnet, dass man einen Wirkstoff der Formel I gemäss Anspruch 1, auf die unerwünschten Pflanzen oder deren Lebensraum einwirken lässt.

8. Verfahren gemäss Anspruch 7 zur selektiven Bekämpfung von monokotylen und dikotylen Unkräutern in Nutzpflanzenkulturen.

9. Verfahren gemäss Anspruch 8 in Soja.

10. Verfahren gemäss Anspruch 8 in Mais.

**Patentansprüche** (für den Vertragsstaat AT)

1. Herbizides Mittel, dadurch gekennzeichnet, dass es neben Träger- und/oder anderen Zuschlagsstoffen als Wirkstoff mindestens einen 3-Halogenalkoxy-4-nitro-2'-chlor-4'-trifluormethyl-diphenyläther der allgemeinen Formel I,

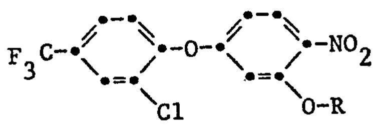

(I)

worin

R Difluormethyl oder 2-Chlor-1,1,2-trifluoräthyl bedeutet, enthält.

2. Mittel gemäss Anspruch 1, dadurch gekennzeichnet, dass es als Wirkstoff 3-(2-Chlor-1,1,2-trifluoräthoxy)-2'-chlor-4'-trifluormethyl-4-nitro-diphenyläther enthält.

3. Mittel gemäss Anspruch 1, dadurch gekennzeichnet, dass es als Wirkstoff 2'-Chlor-3-difluormethoxy-4'-trifluormethyl-4-nitro-diphenyläther enthält.

4. Verfahren zur Herstellung der Wirkstoffe der Formel I, dadurch gekennzeichnet, dass man das Phenoxyphenol der Formel II

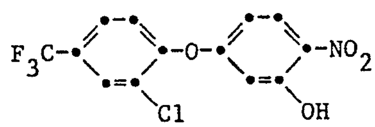

(II)

gegebenenfalls in Gegenwart einer Base, in einem inerten Lösungsmittel, mit einem Halogenalkan der Formel III umsetzt,

Hal—R (III)

worin R die unter Formel I angegebene Bedeutung hat und Hal für Chlor, Brom oder Jod steht.

5. Verfahren zur Herstellung des Wirkstoffs der Formel I, in der R den 2-Chlor-1,1,2-trifluoräthylrest bedeutet, dadurch gekennzeichnet, dass man das Phenoxyphenol der Formel II in Gegenwart einer Base in einem inerten organischen Lösungsmittel, gegebenenfalls unter erhöhtem Druck, mit Chlortrifluoräthan umsetzt.

6. Verfahren zur Bekämpfung unerwünschten Pflanzenwuchses, dadurch gekennzeichnet, dass man einen Wirkstoff der Formel I gemäss Anspruch 1, auf die unerwünschten Pflanzen oder deren Lebensraum einwirken lässt.

7. Verfahren gemäss Anspruch 6 zur selektiven Bekämpfung von monokotylen und dikotylen Unkräutern in Nutzpflanzenkulturen.

8. Verfahren gemäss Anspruch 7 in Soja.

9. Verfahren gemäss Anspruch 7 in Mais.

**Claims** (for the Contracting States : BE, CH, DE, FR, GB, IT, LI, NL)

1. A 3-haloalkoxy-4-nitro-2'-chloro-4'-trifluoromethyl-diphenyl ether of the general formula I

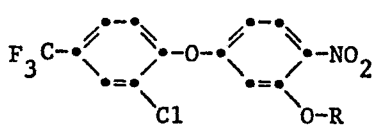

(I)

wherein R is difluoromethyl or 2-chloro-1,1,2-trifluoroethyl.

2. 3-(2-Chloro-1,1,2-trifluoroethoxy)-2'-chloro-4'-trifluoromethyl-4-nitrodiphenyl ether according to claim 1.

3. 2'-Chloro-3-difluoromethoxy-4'-trifluoromethyl-4-nitrodiphenyl ether according to claim 1.

4. A process for the production of a compound of the formula I according to claim 1, which process comprises reacting the phenoxyphenol of the formula II

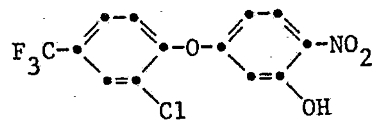

(II)

optionally in the presence of a base and in an inert solvent, with a haloalkane of the formula III

Hal—R (III)

wherein R is as defined for formula I and Hal is chlorine, bromine or iodine.

5. A process for the production of the compound of the formula I according to claim 1, wherein R is the 2-chloro-1,1,2-trifluoroethyl radical, which process comprises reacting the phenoxyphenol of the formula II, in the presence of a base and in an inert organic solvent, optionally under elevated pressure, with chlorotrifluoroethane.

6. A herbicidal composition which contains, as active ingredient, at least one compound according to claim 1, together with carriers and/or other adjuvants.

7. A method of controlling unwanted plant growth, which comprises applying to the unwanted plants or to the locus thereof at least one compound of the formula I according to claim 1.

8. A method according to claim 7 for the selective control of monocot and dicot weeds in crops of useful plants.

9. A method according to claim 8, wherein the crop is soybeans.

10. A method according to claim 8, wherein the crop is maize.

**Claims** (for the Contracting State AT)

1. A herbicidal composition containing, as active ingredient, at least one 3-haloalkoxy-4-nitro-2′-chloro-4′-trifluoromethyldiphenyl ether of the general formula I

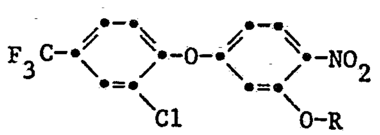

(I)

wherein R is difluoromethyl or 2-chloro-1,1,2-trifluoroethyl, together with carriers and/or other adjuvants.

2. A composition according to claim 1 containing, as active ingredient, 3-(2-chloro-1,1,2-trifluoroethoxy)-2′-chloro-4′-trifluoromethyl-4-nitrodiphenyl ether.

3. A composition according to claim 1 containing, as active ingredient, 2′-chloro-3-difluoromethoxy-4′-trifluoromethyl-4-nitrodiphenyl ether.

4. A process for the production of an active ingredient of the formula I, which process comprises reacting the phenoxyphenol of the formula II

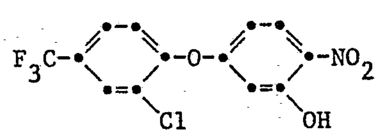

(II)

optionally in the presence of a base and in an inert solvent, with a haloalkane of the formula III

Hal—R (III)

wherein R is as defined for formula I and Hal is chlorine, bromine or iodine.

5. A process for the production of the active ingredient of the formula I, wherein R is the 2-chloro-1,1,2-trifluoroethyl radical, which process comprises reacting the phenoxyphenol of the formula II, in the presence of a base and in an inert organic solvent, optionally under elevated pressure, with chlorotrifluoroethane.

6. A method of controlling unwanted plant growth, which comprises applying to the unwanted plants or to the locus thereof at least one compound of the formula I according to claim 1.

7. A method according to claim 6 for the selective control of monocot and dicot weeds in crops of useful plants.

8. A method according to claim 7, wherein the crop is soybeans.

9. A method according to claim 7, wherein the crop is maize.

**Revendications** (pour les Etats contractants : BE, CH, DE, FR, GB, IT, LI, NL)

1. Ethers 3-halogénoalcoxy-4-nitro-2'-chloro-4'-trifluorométhyl-diphényliques de formule générale I

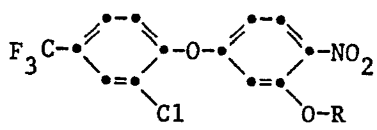 (I)

dans laquelle R représente un groupe difluorométhyl ou 2-chloro-1,1,2-trifluoréthyle.

2. L'éther 3-(2-chloro-1,1,2-trifluoréthoxy)-2'-chloro-4'-trifluorométhyl-4-nitro-diphénylique selon la revendication 1.

3. L'éther 2'-chloro-3-difluorométhoxy-4'-trifluorométhyl-4-nitro-diphénylique selon la revendication 1.

4. Procédé de préparation des composés de formule I selon la revendication 1, caractérisé en ce que l'on fait réagir le phénoxyphénol de formule II

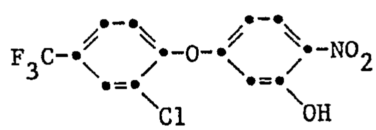 (II)

éventuellement en présence d'une base, dans un solvant inerte, avec un halogénoalcane de formule III

Hal—R (III)

dans laquelle R a la signification indiquée en référence à la formule I et Hal représente le chlore, le brome ou l'iode.

5. Procédé de préparation des composés de formule I selon la revendication 1, dans laquelle R représente le groupe 2-chloro-1,1,2-trifluoréthyle, caractérisé en ce que l'on fait réagir le phénoxyphénol de formule II en présence d'une base dans un solvant organique inerte, éventuellement sous pression, avec le chlorotrifluoréthane.

6. Produit herbicide, caractérisé en ce qu'il contient en tant que substance active, avec des véhicules et/ou d'autres additifs, au moins un composé selon la revendication 1.

7. Procédé pour combattre les croissances de végétaux indésirables, caractérisé en ce qu'on fait réagir une substance active de formule I selon la revendication 1 sur les végétaux indésirables ou leur habitat.

8. Procédé selon la revendication 7 pour la lutte sélective contre les mauvaises herbes monocotilédones et dicotilédones dans les cultures de végétaux utiles.

9. Procédé selon la revendication 8, dans les cultures de soya.

10. Procédé selon la revendication 8, dans la culture de maïs.

**Revendications** (pour l'Etat contractant AT)

1. Produit herbicide caractérisé en ce qu'il contient en tant que substance active, avec des véhicules et/ou d'autres additifs, au moins un éther 3-halogénoalcoxy-4-nitro-2'-chloro-4'-trifluorométhyl-diphénylique de formule générale I

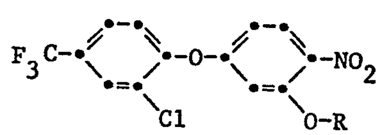 (I)

dans laquelle R représente un groupe difluorométhyle ou 2-chloro-1,1,2-trifluoréthyle.

2. Produit selon la revendication 1, caractérisé en ce qu'il contient en tant que substance active l'éther 3-(2-chloro-1,1,2-trifluoréthoxy)-2'-chloro-4'-trifluorométhyl-4-nitro-diphénylique.

3. Produit selon la revendication 1, caractérisé en ce qu'il contient en tant que substance active l'éther 2'-chloro-3-difluorométhoxy-4'-trifluorométhyl-4-nitrodiphénylique.

4. Procédé de préparation des substances actives de formule I, caractérisé en ce que l'on fait réagir le phénoxyphénol de formule II

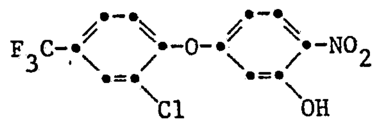

(II)

éventuellement en présence d'une base, dans un solvant inerte, avec un halogénoalcane de formule III

Hal—R (III)

dans laquelle R a la signification indiquée en référence à la formule I et Hal représente le chlore, le brome ou l'iode.

5. Procédé de préparation de la substance active de formule I dans laquelle R représente le groupe 2-chloro-1,1,2-trifluoréthyle, caractérisé en ce que l'on fait réagir le phénoxyphénol de formule II en présence d'une base, dans un solvant organique inerte, éventuellement sous pression, avec le chlorotrifluoréthane.

6. Procédé pour combattre les croissances de végétaux indésirables, caractérisé en ce que l'on fait agir une substance active de formule I selon la revendication 1 sur les végétaux indésirables ou leur habitat.

7. Procédé selon la revendication 6, pour la lutte sélective contre les mauvaises herbes monocotilédones et dicotilédones dans les cultures de végétaux utiles.

8. Procédé selon la revendication 7, dans les cultures de soya.

9. Procédé selon la revendication 7, dans les cultures de maïs.